# EUROPEAN PATENT APPLICATION

(11) **EP 3 441 016 A1**
(43) Date of publication of application: **13.02.2019**
(21) Application number: 18187640.0
(22) Date of filing: 07.08.2018
(51) Int. Cl.: A61B 17/128, A61B 17/068, A61B 17/10, A61B 17/115

(54) **GEARED ACTUATION MECHANISM AND SURGICAL CLIP APPLIER INCLUDING THE SAME**

(30) Priority: 08.08.2017 US 201762542330 P; 10.07.2018 US 201816030971
(71) Applicant: Covidien LP, Mansfield, MA 02048 (US)
(72) Inventor: BARIL, Jacob C., White Plains, NY New York 10605 (US); DININO, Matthew A., Newington, CT Connecticut 06111 (US)
(74) Representative: Maschio, Antonio

(57) **Abstract**

An actuation mechanism, handle assembly including the same, and surgical clip applier including the same are disclosed. The actuation mechanism includes a drive bar having an output gear rack associated therewith, a trigger having an input gear associated therewith, and first and second intermediate gears each including input and output portions having different radiuses. The input gear is disposed in meshed engagement with the input portion of the first intermediate gear, the output portion of the first intermediate gear is disposed in meshed engagement with the input portion of the second intermediate gear, and the output portion of the second intermediate gear is disposed in meshed engagement with the gear rack such that pivoting of the trigger from an un-actuated to an actuated position moves the drive bar from a proximal to a distal position to fire a surgical clip(s). A consistent mechanical advantage is provided throughout actuation.

## Description

### CROSS-REFERENCE TO RELATED APPLICATIONS

This application claims the benefit of and priority to U.S. Provisional Patent Application No. 62/542,330 filed August 8, 2017, the entire disclosure of which is incorporated by reference herein.

### BACKGROUND

### Technical Field

The present disclosure relates to surgical clip appliers. More particularly, the present disclosure relates to geared actuation mechanisms for surgical clip appliers and surgical clip appliers including the same.

### Description of Related Art

Surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures.

Endoscopic surgical clip appliers having various sizes (e.g., diameters), that are configured to apply a variety of diverse surgical clips, are also known in the art, and are capable of applying a single or multiple surgical clips during an entry to the body cavity. Such surgical clips are typically fabricated from a biocompatible material and are usually compressed over tissue. Once applied to tissue, the compressed surgical clip terminates the flow of fluid therethrough.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

Provided in accordance with aspects of the present disclosure is an actuation mechanism for a surgical clip applier. The actuation mechanism includes a drive bar movable from a proximal position to a distal position to fire at least one surgical. The drive bar includes an output gear rack associated therewith. The actuation mechanism further includes a trigger pivotable from an un-actuated position to an actuated position and including an input gear associated therewith, and first and second intermediate gears each including input and output portions defining different radiuses. The input gear is disposed in meshed engagement with the input portion of the first intermediate gear, the output portion of the first intermediate gear is disposed in meshed engagement with the input portion of the second intermediate gear, and the output portion of the second intermediate gear is disposed in meshed engagement with the gear rack. As such, pivoting of the trigger from the un-actuated position to the actuated position moves the drive bar from the proximal position to the distal position. A consistent mechanical advantage is provided throughout pivoting of the trigger from the un-actuated position to the actuated position.

In an aspect of the present disclosure, the mechanical advantage is 1.65 to 1.80. In aspects, the mechanical advantage is about 1.73.

In another aspect of the present disclosure, the trigger is pivotable from the un-actuated position to the actuated position through an input rotation of 35 degrees to 45 degrees. In aspects, the trigger is pivotable from the un-actuated position to the actuated position through an input rotation of 40 degrees.

In another aspect of the present disclosure, the drive bar is movable from the proximal position to the distal position a stoke length of 1.0 inch to 1.4 inches. In aspects, the drive bar is movable from the proximal position to the distal position a stoke length of 1.2 inches.

A handle assembly of a surgical clip applier provided in accordance with aspects of the present disclosure includes a housing defining a body portion and a fixed handle portion extending from the body portion and an actuation mechanism such as the actuation mechanism according to any of the above-noted aspects or any of the other aspects detailed herein.

In aspects, the housing is configured to receive an elongated assembly supporting an end effector assembly at a distal end portion thereof. The drive bar is configured to operably interface with an actuation member of the elongated assembly.

A surgical clip applier provided in accordance with aspects of the present disclosure includes a handle assembly and an elongated assembly extending distally from the handle assembly and supporting an end effector assembly at a distal end portion thereof. The elongated assembly includes an actuation member movable to fire at least one surgical clip from the end effector assembly. The handle assembly includes a housing defining a body portion and a fixed handle portion extending from the body portion. The handle assembly further includes an actuation mechanism such as the actuation mechanism according to any of the above-noted aspects or any of the other aspects detailed herein.

In aspects, the elongated assembly is releasably engagable with the handle assembly.

### BRIEF DESCRIPTION OF THE DRAWINGS

Aspects and features of the presently-disclosed geared actuation mechanisms for surgical clip appliers and clip surgical clip appliers including the same are described in detail with reference to the drawing figures wherein like reference numerals identify similar or identical structural elements and:
FIG. 1 is a front, perspective view of a surgical clip applier provided in accordance with the present disclosure including a handle assembly having an elongated assembly engaged therewith;
FIG. 2 is front, perspective view of the surgical clip applier of FIG. 1 with the elongated assembly removed from the handle assembly;
FIG. 3 is a side, perspective view of a distal end portion of the elongated assembly of FIGS. 1 and 2;
FIG. 4 is a side, perspective view of a distal end portion of another elongated assembly configured for use with the surgical clip applier of FIG. 1;
FIG. 5 is an enlarged, longitudinal, cross-sectional view of a portion of the handle assembly of the surgical clip applier of FIG. 1; and
FIG. 6 is an exploded, side view of an actuation mechanism of the handle assembly of the surgical clip applier of FIG. 1.

### DETAILED DESCRIPTION

The present disclosure provides geared actuation mechanisms for surgical clip appliers and surgical clip appliers including the same.

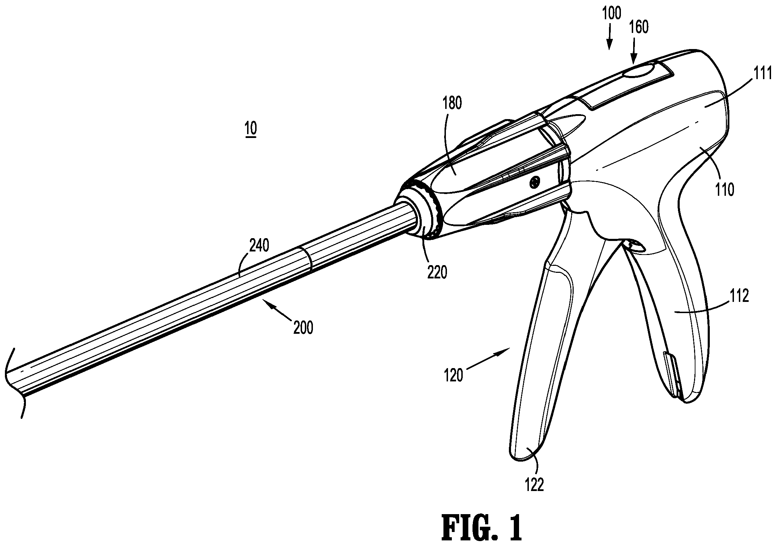

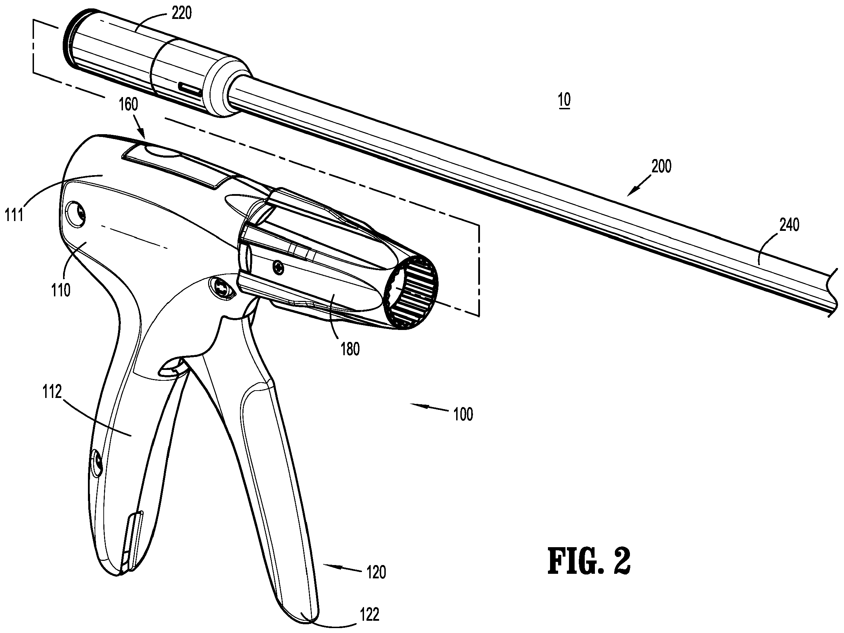

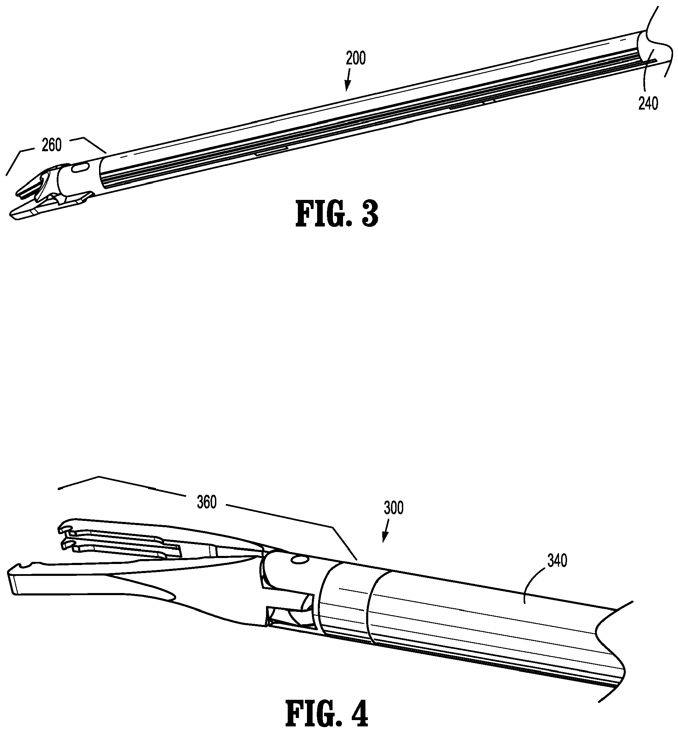

Turning to FIGS. 1-2, a surgical clip applier embodying the aspects and features of the present disclosure is shown generally identified by reference numeral 10. Surgical clip applier 10 generally includes a handle assembly 100 and a plurality of elongated assemblies 200, 300 (FIG. 4) selectively connectable to handle assembly 100. Handle assembly 100 is configured to operate each of the plurality of elongated assemblies 200, 300 (FIG. 4) upon connection thereto, and may be configured as a sterilizable, reusable component such that handle assembly 100 may be repeatedly used with different and/or additional elongated assemblies 200, 300 (FIG. 4) during the course of one or more surgical procedures. The elongated assemblies 200, 300 (FIG. 4) may be configured as single-use disposable components, limited-use disposable components, or reusable components, depending upon a particular purpose and/or the configuration of the particular elongated assembly. In either configuration, the need for multiple handle assemblies 100 is obviated and, instead, the surgeon need only select an appropriate elongated assembly 200, 300 (FIG. 4) and connect that elongated assembly to handle assembly 100 in preparation for use.

Handle assembly 100 generally includes a housing 110, an actuation mechanism 120 operably associated with housing 110, a latch assembly 160 operably associated with housing 110, a rotating receiver assembly 180 operably coupled to a distal portion of housing 110, and, in embodiments, a ratchet mechanism (not shown) operably disposed within housing 110. Housing 110 supports and/or encloses the operating components of handle assembly 100 and is detailed below. Actuation mechanism 120 is configured to enable selective firing of one or more surgical clips (not shown) from the end effector of the attached elongated assembly, as also detailed below.

Latch assembly 160 is configured to facilitate releasable locking engagement of the elongated assembly with handle assembly 100. Rotating receiver assembly 180 is configured to receive a proximal end portion of the elongated assembly and to enable selective rotation thereof relative to housing 110. The ratchet mechanism is configured to enable ratcheting actuation of actuation mechanism 120, when an elongated assembly configured for ratcheting actuation is connected to handle assembly 100. Details of latch assembly 160, rotating receiver assembly 180, and the ratchet mechanism can be found in International Application No. PCT/CN2016/096666, filed on August 26, 2016, the entire contents of which is hereby incorporated herein by reference. Alternatively or additionally, latch assembly 160, rotating receiver assembly 180, and/or the ratchet mechanism may be configured as detailed in International Application No. PCT/CN2016/071178, filed on January 18, 2016, the entire contents of which is also hereby incorporated herein by reference.

With additional reference to FIGS. 3 and 4, as noted above, handle assembly 100 is configured for use with different elongated assemblies such as, for example, elongated assembly 200 (FIGS. 1-3) and elongated assembly 300 (FIG. 4). Handle assembly 100, more specifically, is configured for both ratcheting use, e.g., in connection with elongated assembly 200 (FIGS. 1-3), and non-ratcheting use, e.g., in connection with elongated assembly 300 (FIG. 4). Elongated assemblies 200, 300 are described briefly below. A more detailed discussion of elongated assemblies, e.g., elongated assemblies 200, 300, configured for use with handle assembly 100 can be found in International Application Nos. PCT/CN2016/096666 and/or PCT/CN2016/071178, previously incorporated by reference herein in their entireties, and additionally or alternatively as in International Application No. PCT/CN2015/091603, filed on October 10, 2015, the entire contents of which is likewise hereby incorporated herein by reference.

Referring to FIGS. 1-3, elongated assembly 200 is configured for ratcheting use and generally includes a proximal hub 220, an elongated shaft 240 extending distally from proximal hub 220, an end effector assembly 260 disposed towards a distal end portion of elongated shaft 240, and an inner drive assembly (not shown) operably coupled between handle assembly 100 and end effector assembly 260 when elongated assembly 200 is engaged with handle assembly 100 to enable the sequential firing of at least one surgical clip (not shown) about tissue. End effector assembly 260 of elongated assembly 200 may be configured to fire surgical clips similar to those shown and described in U.S. Patent Nos. 7,819,886 or 7,905,890, the entire contents of each of which is hereby incorporated herein by reference.

Referring to FIG. 4, elongated assembly 300 is configured for non-ratcheting use and generally includes a proximal hub (not shown), an elongated shaft 340 extending distally from the proximal hub, an end effector assembly 360 disposed towards a distal end portion of elongated shaft 340, and an inner drive assembly (not shown) operably coupled between handle assembly 100 and end effector assembly 360 when elongated assembly 300 is engaged with handle assembly 100 to enable grasping and/or manipulation of tissue, retrieval of a surgical clip, and firing of the surgical clip about tissue. It is contemplated that end effector assembly 360 of elongated assembly 300 may be configured to fire surgical clips similar to those shown and described in U.S. Patent No. 4,834,096, the entire contents of which is hereby incorporated herein by reference.

Referring generally to FIGS. 1-4, although exemplary elongated assemblies 200, 300 configured for ratcheting and non-ratcheting use, respectively, are detailed above, it is contemplated that various other elongated assemblies for performing various different surgical tasks and/or having various different configurations suitable for ratcheting or non-ratcheting use may likewise be utilized with handle assembly 100.

Turning to FIGS. 1, 2, and 5, housing 110 of handle assembly 100 defines a body portion 111 and a fixed handle portion 112 depending from body portion 111. Body portion 111 of housing 110 includes an internal pivot post 114 extending transversely within body portion 111, a retention post 116 disposed towards a proximal end of body portion 111 and likewise extending transversely within body portion 111, and a distal opening 118 through which a proximal end portion of an actuation member "A" of an elongated assembly, e.g., elongated assembly 200 (FIGS. 1-3) or elongated assembly 300 (FIG. 4), extends when the elongated assembly is engaged with handle assembly 100.

Actuation mechanism 120 is operably supported by housing 110 and includes a trigger 122, a drive bar 130, a biasing member 140, and a gear assembly 150. Trigger 122 includes a grasping portion 123, an intermediate pivot portion 124, and a proximal extension 125. Grasping portion 123 of trigger 122 extends downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture 126 that is configured to receive pivot post 114 of housing 110 so as to enable pivoting of trigger 122 about pivot post 114 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

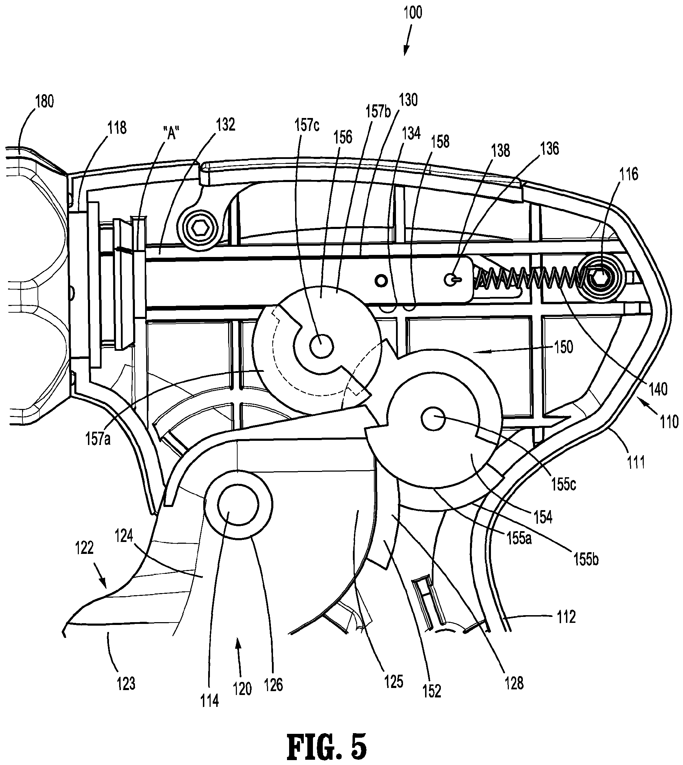

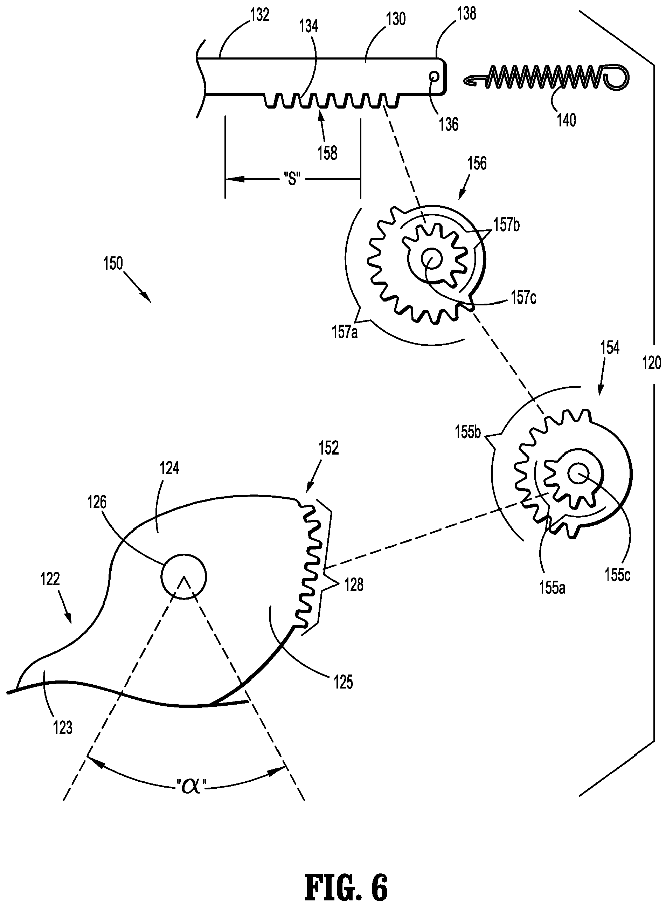

Proximal extension 125 of trigger 122 is disposed on an opposite side of intermediate pivot portion 124 and, thus, pivot post 114, as compared to grasping portion 123 of trigger 122. As such, pivoting of grasping portion 123 to rotate in one direction, e.g., proximally towards fixed handle portion 112, pivots proximal extension 125 to rotate in the opposite direction, e.g., distally. Proximal extension 125 of trigger 122 defines an arcuate gear portion 128 (FIGS. 5 and 6) that functions as an input gear 152 of gear assembly 150, as detailed below.

The actuation member "A" of the elongated assembly, engaged with handle assembly 100, is selectively translatable in response to actuation of actuation mechanism 120 to fire a surgical clip (not shown) supported at the end effector assembly of the elongated assembly about tissue. More specifically, drive bar 130 is slidably disposed within body portion 111 of housing 110 in longitudinal alignment with the actuation member "A" such that distal sliding of drive bar 130 through body portion 111 of housing urges a distal end portion 132 of drive bar 130 into contact with actuation member "A" to thereby translate actuation member "A" distally, e.g., to fire a surgical clip supported at the end effector assembly of the elongated assembly.

With additional reference to FIG. 6, drive bar 130 defines a linear gear rack 134 extending along at least a portion of an underside surface thereof. Linear gear rack 134 functions as an output gear 158 of gear assembly 150, as detailed below. Drive bar 130, as detailed above, is slidable through body portion 111 of housing to urge distal end portion 132 of drive bar 130 into actuation member "A" to translate actuation member "A" distally to thereby to fire a surgical clip supported at the end effector assembly of the elongated assembly. Drive bar 130, more specifically, is slidable from an un-actuated, proximal position to an actuated, distal position in order to fire a surgical clip supported at the end effector assembly of the elongated assembly. Biasing member 140 is engaged at a first, proximal end portion thereof to retention post 116 of body portion 111 of housing 110 and at a second, distal end portion thereof through an aperture 136 defined through a proximal end portion 138 of drive bar 130. In this manner, biasing member 140 biases drive bar 130 towards the un-actuated, proximal position.

Gear assembly 150 includes, as noted above, an input gear 152 (defined by arcuate gear portion 128 of proximal extension 125 of trigger 122) and an output gear 158 (defined by linear gear rack 134 of drive bar 130). Gear assembly 150 further includes a first intermediate gear 154 and a second intermediate gear 156.

First intermediate gear 154 is configured as a compound gear including an arcuate input gear portion 155a (extending a partial or full circumference) and an arcuate output gear portion 155b (extending a partial or full circumference). Arcuate input gear portion 155a of first intermediate gear 154 is disposed in meshed engagement with input gear 152, e.g., arcuate gear portion 128 of proximal extension 125 of trigger 122. First intermediate gear 154 is rotatably mounted about a pin 155c that is fixed relative to and extends transversely through body portion 111 of housing 110. In embodiments, arcuate input gear portion 155a defines an arc having a radius different from a radius of the arc defined by arcuate output gear portion 155b. In embodiments, the radius of arcuate input gear portion 155a is less than the radius of arcuate output gear portion 155b, although other configurations are also contemplated.

Second intermediate gear 156, similar to first intermediate gear 154, is configured as a compound gear including an arcuate input gear portion 157a (extending a partial or full circumference) and an arcuate output gear portion 157b (extending a partial or full circumference). Arcuate input gear portion 157a of second intermediate gear 156 is disposed in meshed engagement with arcuate output gear portion 155b of first intermediate gear 154. Arcuate output gear portion 157b of second intermediate gear 156 is disposed in meshed engagement with output gear 158, e.g., linear gear rack 134 of drive bar 130. Second intermediate gear 156 is rotatably mounted about a pin 157c that is fixed relative to and extends transversely through body portion 111 of housing 110. In embodiments, arcuate input gear portion 157a defines an arc having a radius different from a radius of the arc defined by arcuate output gear portion 157b. In embodiments, the radius of arcuate input gear portion 157a is greater than the radius of arcuate output gear portion 157b, although other configurations are also contemplated.

With continued reference to FIGS. 1, 2, 5, and 6, and to FIGS. 5 and 6 in particular, as a result of the above-detailed configuration, pivoting of trigger 122 from the un-actuated position towards the actuated position rotates input gear 152 in a counter-clockwise direction (as viewed from the orientation shown in FIGS. 5 and 6). Counter-clockwise rotation of input gear 152, in turn, rotates first intermediate gear 154 in a clockwise direction (as viewed from the orientation shown in FIGS. 5 and 6), due to the meshed engagement between arcuate gear portion 128 of input gear 152 and arcuate input gear portion 155a of first intermediate gear 154. Clockwise rotation of first intermediate gear 154 rotates second intermediate gear 156 in a counter-clockwise direction (as viewed from the orientation shown in FIGS. 5 and 6), due to the meshed engagement between arcuate output gear portion 155b of first intermediate gear 154 and arcuate input gear portion 157a of second intermediate gear 156. Counter-clockwise rotation of second intermediate gear 156, in turn, translates drive bar 130 distally through body portion 111 of housing 110 due to the meshed engagement between arcuate output gear portion 157b of second intermediate gear 156 and linear gear rack 134 of drive bar 130.

The above-detailed distal advancement of drive bar 130 in response to actuation of trigger 122 occurs against the biasing of biasing member 140. As such, upon release of trigger 122, the bias of biasing member 140 pulls drive bar 130 proximally, thereby urging second intermediate gear 156 to rotate clockwise (as viewed from the orientation shown in FIGS. 5 and 6) which, in turn, rotates first intermediate gear 154 counter-clockwise (as viewed from the orientation shown in FIGS. 5 and 6) and, thus, first gear 152 clockwise (as viewed from the orientation shown in FIGS. 5 and 6), thereby returning trigger 122 towards the un-actuated position.

The above-detailed actuation mechanism 120 provides a compact configuration to enable use with reposable surgical clip applier 10 (FIGS. 1-2), wherein elongated assemblies, e.g., elongated assemblies 200, 300 (FIGS. 3 and 4), are releasably engagable with handle assembly 100 of reposable surgical clip applier 10 (FIGS. 1-2); however, actuation mechanism 120 is equally applicable for use with integrated surgical clip appliers and other configurations of surgical clip appliers.

The above-detailed actuation mechanism 120 is configured to provide an appropriate input rotation "α" (FIG. 6), e.g., the angle through which the user pivots trigger 122 between the un-actuated and actuated positions; an appropriate output stroke length "S" (FIG. 6), e.g., the distance drive bar 130 is moved through body portion 111 of housing 110; and to maintain an appropriate and consistent mechanical advantage throughout actuation, e.g., throughout the entire input rotation "α" and output stroke length "S." In embodiments, the input rotation "α" is 35 to 45 degrees, in embodiments, 37.5 degrees to 42.5 degrees, and, in embodiments, about 40 degrees (wherein the "about" takes into account manufacturing, material, environmental, and other tolerances). In embodiments, the output stroke length "S" is 1.0 inch to 1.4 inches, in embodiments, 1.1 inches to 1.3 inches, and, in embodiments, about 1.2 inches (wherein the "about" takes into account manufacturing, material, environmental, and other tolerances). In embodiments, the mechanical advantage is 1.65 to 1.8, in embodiments, 1.70 to 1.75, and, in embodiments, about 1.73 (wherein the "about" takes into account manufacturing, material, environmental, and other tolerances). The relative radiuses of arcuate gear portion 128, arcuate input gear portion 155a, arcuate output gear portion 155b, arcuate input gear portion 157a, and arcuate output gear portion 157b as selected so as to achieve the above, or other suitable input rotation "α," output stroke length "S," and/or mechanical advantage.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. An actuation mechanism for a surgical clip applier, the actuation mechanism comprising:
   a drive bar movable from a proximal position to a distal position to fire at least one surgical clip, the drive bar including an output gear rack associated therewith;
   a trigger pivotable from an un-actuated position to an actuated position, the trigger including an input gear associated therewith;
   a first intermediate gear including an input portion and an output portion, the input and output portions of the first intermediate gear defining different radiuses; and
   a second intermediate gear including an input portion and an output portion, the input and output portions of the second intermediate gear defining different radiuses,
   wherein the input gear is disposed in meshed engagement with the input portion of the first intermediate gear, the output portion of the first intermediate gear is disposed in meshed engagement with the input portion of the second intermediate gear, and the output portion of the second intermediate gear is disposed in meshed engagement with the gear rack such that pivoting of the trigger from the un-actuated position to the actuated position moves the drive bar from the proximal position to the distal position, and
   wherein a consistent mechanical advantage is provided throughout pivoting of the trigger from the un-actuated position to the actuated position.
2. The actuation mechanism according to paragraph 1, wherein the mechanical advantage is 1.65 to 1.80.
3. The actuation mechanism according to paragraph 1, wherein the mechanical advantage is about 1.73.
4. The actuation mechanism according to paragraph 1, wherein the trigger is pivotable from the un-actuated position to the actuated position through an input rotation of 35 degrees to 45 degrees.
5. The actuation mechanism according to paragraph 1, wherein the trigger is pivotable from the un-actuated position to the actuated position through an input rotation of 40 degrees.
6. The actuation mechanism according to paragraph 1, wherein the drive bar is movable from the proximal position to the distal position a stoke length of 1.0 inch to 1.4 inches.
7. The actuation mechanism according to paragraph 1, wherein the drive bar is movable from the proximal position to the distal position a stoke length of 1.2 inches.
8. A handle assembly of a surgical clip applier, comprising:
   a housing defining a body portion and a fixed handle portion extending from the body portion;
   a drive bar slidably supported within the body portion of the housing and movable relative thereto from a proximal position to a distal position to fire at least one surgical clip, the drive bar including an output gear rack associated therewith;
   a trigger pivotably connected to the housing and movable relative to the fixed handle portion thereof from an un-actuated position to an actuated position, the trigger including an input gear associated therewith;
   a first intermediate gear rotatably mounted within the housing and including an input portion and an output portion, the input and output portions of the first intermediate gear defining different radiuses; and
   a second intermediate gear rotatably mounted within the housing and including an input portion and an output portion, the input and output portions of the second intermediate gear defining different radiuses,
   wherein the input gear is disposed in meshed engagement with the input portion of the first intermediate gear, the output portion of the first intermediate gear is disposed in meshed engagement with the input portion of the second intermediate gear, and the output portion of the second intermediate gear is disposed in meshed engagement with the gear rack such that pivoting of the trigger from the un-actuated position to the actuated position moves the drive bar from the proximal position to the distal position, and
   wherein a consistent mechanical advantage is provided throughout pivoting of the trigger from the un-actuated position to the actuated position.
9. The handle assembly according to paragraph 8, wherein the housing is configured to receive an elongated assembly supporting an end effector assembly at a distal end portion thereof, and wherein the drive bar is configured to operably interface with an actuation member of the elongated assembly.
10. The handle assembly according to paragraph 8, wherein the mechanical advantage is 1.65 to 1.80.
11. The handle assembly according to paragraph 8, wherein the trigger is pivotable from the un-actuated position to the actuated position through an input rotation of 35 degrees to 45 degrees.
12. The handle assembly according to paragraph 8, wherein the drive bar is movable from the proximal position to the distal position a stoke length of 1.0 inch to 1.4 inches.
13. A surgical clip applier, comprising:
   a handle assembly; and
   an elongated assembly extending distally from the handle assembly and supporting an end effector assembly at a distal end portion thereof, elongated assembly including an actuation member movable to fire at least one surgical clip from the end effector assembly,
   wherein the handle assembly includes:
      a housing defining a body portion and a fixed handle portion extending from the body portion;
      a drive bar slidably supported within the body portion of the housing and movable relative thereto from a proximal position to a distal position, the drive bar configured to interface with the actuation member such that movement of the drive bar from the proximal position to the distal position moves the actuation member to fire the at least one surgical clip, the drive bar including an output gear rack associated therewith;
      a trigger pivotably connected to the housing and movable relative to the fixed handle portion thereof from an un-actuated position to an actuated position, the trigger including an input gear associated therewith;
      a first intermediate gear rotatably mounted within the housing and including an input portion and an output portion, the input and output portions of the first intermediate gear defining different radiuses; and
      a second intermediate gear rotatably mounted within the housing and including an input portion and an output portion, the input and output portions of the second intermediate gear defining different radiuses,
      wherein the input gear is disposed in meshed engagement with the input portion of the first intermediate gear, the output portion of the first intermediate gear is disposed in meshed engagement with the input portion of the second intermediate gear, and the output portion of the second intermediate gear is disposed in meshed engagement with the gear rack such that pivoting of the trigger from the un-actuated position to the actuated position moves the drive bar from the proximal position to the distal position, and
      wherein a consistent mechanical advantage is provided throughout pivoting of the trigger from the un-actuated position to the actuated position.
14. The surgical clip applier according to paragraph 13, wherein the mechanical advantage is 1.65 to 1.80.
15. The surgical clip applier according to paragraph 13, wherein the mechanical advantage is about 1.73.
16. The surgical clip applier according to paragraph 13, wherein the trigger is pivotable from the un-actuated position to the actuated position through an input rotation of 35 degrees to 45 degrees.
17. The surgical clip applier according to paragraph 13, wherein the trigger is pivotable from the un-actuated position to the actuated position through an input rotation of 40 degrees.
18. The surgical clip applier according to paragraph 13, wherein the drive bar is movable from the proximal position to the distal position a stoke length of 1.0 inch to 1.4 inches.
19. The surgical clip applier according to paragraph 13, wherein the drive bar is movable from the proximal position to the distal position a stoke length of 1.2 inches.
20. The surgical clip applier according to paragraph 13, wherein the elongated assembly is releasably engagable with the handle assembly.

## Claims

1. An actuation mechanism for a surgical clip applier, the actuation mechanism comprising:
a drive bar movable through a stroke having a first stroke portion and a second stroke portion to fire at least one surgical clip, the drive bar including an output gear rack associated therewith;
a trigger pivotable from an un-actuated position to an actuated position;
a first intermediate gear including an input portion and an output portion, the input portion operably coupled to the trigger such that pivoting of the trigger from the un-actuated position to the actuated position rotates the first intermediate gear; and
a second intermediate gear including an input portion and an output portion, the output portions of the first and second intermediate gears defining different diameters, the input portion of the second intermediate gear operably coupled to the trigger such that pivoting of the trigger from the un-actuated position to the actuated position rotates the second intermediate gear,
wherein, in the first portion of the stroke length, the output portion of the first intermediate gear is disposed in meshed engagement with the gear rack and the output portion of the second intermediate gear is disengaged from the gear rack such that the first intermediate gear drives the drive bar along the first portion of the stroke length in response to pivoting of the trigger,
wherein, in the second portion of the stroke length, the output portion of the second intermediate gear is disposed in meshed engagement with the gear rack and the output portion of the first intermediate gear is disengaged from the gear rack such that the second intermediate gear drives the drive bar along the second portion of the stroke length in response to pivoting of the trigger, and
wherein a different mechanical advantage is provided in the first portion of the stroke length as compared to the second portion of the stroke length.

2. The actuation mechanism according to claim 1, wherein the mechanical advantage provided in the first portion of the stroke length is less than the mechanical advantage provided in the second portion of the stroke length.

3. The actuation mechanism according to claim 1 or claim 2, wherein the first portion of the stroke length is greater than the second portion of the stroke length.

4. The actuation mechanism according to any preceding claim, wherein the trigger is pivotable through an input rotation from the un-actuated position to the actuated position, the input rotation including a first portion corresponding to the first portion of the stroke length and a second portion corresponding to the second portion of the stroke length; preferably wherein the first portion of the input rotation is less than the second portion of the input rotation; preferably wherein the first portion of the stroke length is greater than the second portion of the stroke length.

5. The actuation mechanism according to any preceding claim, further comprising:
an input gear associated with the trigger; and
a transmission gear including an input portion and an output portion, the input portion of the transmission gear disposed in meshed engagement with the input gear and the output portion of the transmission gear disposed in meshed engagement with the input portions of the first and second intermediate gears.

6. A handle assembly of a surgical clip applier, comprising:
a housing defining a body portion and a fixed handle portion extending from the body portion;
a drive bar slidably supported within the body portion of the housing and movable relative thereto through a stroke having a first stroke portion and a second stroke portion to fire at least one surgical clip, the drive bar including an output gear rack associated therewith;
a trigger pivotably connected to the housing and movable relative to the fixed handle portion thereof from an un-actuated position to an actuated position;
a first intermediate gear including an input portion and an output portion, the input portion operably coupled to the trigger such that pivoting of the trigger from the un-actuated position to the actuated position rotates the first intermediate gear; and
a second intermediate gear including an input portion and an output portion, the output portions of the first and second intermediate gears defining different diameters, the input portion of the second intermediate gear operably coupled to the trigger such that pivoting of the trigger from the un-actuated position to the actuated position rotates the second intermediate gear,
wherein, in the first portion of the stroke length, the output portion of the first intermediate gear is disposed in meshed engagement with the gear rack and the output portion of the second intermediate gear is disengaged from the gear rack such that the first intermediate gear drives the drive bar along the first portion of the stroke length in response to pivoting of the trigger,
wherein, in the second portion of the stroke length, the output portion of the second intermediate gear is disposed in meshed engagement with the gear rack and the output portion of the first intermediate gear is disengaged from the gear rack such that the second intermediate gear drives the drive bar along the second portion of the stroke length in response to pivoting of the trigger, and
wherein a different mechanical advantage is provided in the first portion of the stroke length as compared to the second portion of the stroke length.

7. The handle assembly according to claim 6, wherein the mechanical advantage provided in the first portion of the stroke length is less than the mechanical advantage provided in the second portion of the stroke length; preferably wherein the first portion of the stroke length is greater than the second portion of the stroke length; and/or wherein the trigger is pivotable through an input rotation from the un-actuated position to the actuated position, the input rotation including a first portion corresponding to the first portion of the stroke length and a second portion corresponding to the second portion of the stroke length,
wherein the first portion of the input rotation is less than the second portion of the input rotation, and the first portion of the stroke length is greater than the second portion of the stroke length.

8. The handle assembly according to claim 7, further comprising:
an input gear associated with the trigger; and
a transmission gear including an input portion and an output portion, the input portion of the transmission gear disposed in meshed engagement with the input gear and the output portion of the transmission gear disposed in meshed engagement with the input portions of the first and second intermediate gears.

9. The handle assembly according to claim 7 or claim 8, wherein the housing is configured to receive an elongated assembly supporting an end effector assembly at a distal end portion thereof, and wherein the drive bar is configured to operably interface with an actuation member of the elongated assembly.

10. A surgical clip applier, comprising:
a handle assembly; and
an elongated assembly extending distally from the handle assembly and supporting an end effector assembly at a distal end portion thereof, elongated assembly including an actuation member movable to fire at least one surgical clip from the end effector assembly,
wherein the handle assembly includes:
a housing defining a body portion and a fixed handle portion extending from the body portion;
a drive bar slidably supported within the body portion of the housing and movable relative thereto through a stroke having a first stroke portion and a second stroke portion to fire at least one surgical clip, the drive bar including an output gear rack associated therewith;
a trigger pivotably connected to the housing and movable relative to the fixed handle portion thereof from an un-actuated position to an actuated position;
a first intermediate gear including an input portion and an output portion, the input portion operably coupled to the trigger such that pivoting of the trigger from the un-actuated position to the actuated position rotates the first intermediate gear; and
a second intermediate gear including an input portion and an output portion, the output portions of the first and second intermediate gears defining different diameters, the input portion of the second intermediate gear operably coupled to the trigger such that pivoting of the trigger from the un-actuated position to the actuated position rotates the second intermediate gear,
wherein, in the first portion of the stroke length, the output portion of the first intermediate gear is disposed in meshed engagement with the gear rack and the output portion of the second intermediate gear is disengaged from the gear rack such that the first intermediate gear drives the drive bar along the first portion of the stroke length in response to pivoting of the trigger,
wherein, in the second portion of the stroke length, the output portion of the second intermediate gear is disposed in meshed engagement with the gear rack and the output portion of the first intermediate gear is disengaged from the gear rack such that the second intermediate gear drives the drive bar along the second portion of the stroke length in response to pivoting of the trigger, and
wherein a different mechanical advantage is provided in the first portion of the stroke length as compared to the second portion of the stroke length.

11. The surgical clip applier according to claim 10, wherein the mechanical advantage provided in the first portion of the stroke length is less than the mechanical advantage provided in the second portion of the stroke length.

12. The surgical clip applier according to claim 10 or claim 11, wherein the first portion of the stroke length is greater than the second portion of the stroke length.

13. The surgical clip applier according to any of claims 10 to 12, wherein the trigger is pivotable through an input rotation from the un-actuated position to the actuated position, the input rotation including a first portion corresponding to the first portion of the stroke length and a second portion corresponding to the second portion of the stroke length; preferably wherein the first portion of the input rotation is less than the second portion of the input rotation, and the first portion of the stroke length is greater than the second portion of the stroke length.

14. The surgical clip applier according to any of claims 10 to 13, further comprising:
an input gear associated with the trigger; and
a transmission gear including an input portion and an output portion, the input portion of the transmission gear disposed in meshed engagement with the input gear and the output portion of the transmission gear disposed in meshed engagement with the input portions of the first and second intermediate gears.

15. The surgical clip applier according to any of claims 10 to 14, wherein the elongated assembly is releasably engagable with the handle assembly.
